# EUROPÄISCHE PATENTSCHRIFT

(11) **EP 1 035 413 B1**
(45) Veröffentlichungstag und Bekanntmachung des Hinweises auf die Patenterteilung: **10.03.2004**
(21) Anmeldenummer: 99125089.5
(22) Anmeldetag: 16.12.1999
(51) Int. Cl.: G01N 33/36, G01N 27/22

(54) **Vorrichtung zur automatischen Prüfung der Gleichmässigkeit von textilem Prüfgut**
Apparatus for automatically testing the regularity of textile samples
Appareil pour tester automatiquement la régularité d'échantillons textiles

(30) Priorität: 25.02.1999 DE 19908236
(43) Veröffentlichungstag der Anmeldung: 13.09.2000
(73) Patentinhaber: TEXTECHNO HERBERT STEIN GMBH & CO. KG, 41066 Mönchengladbach (DE); KEISOKKI KOGYO CO., LTD., Amagasaki-shi, Hyogo-ken (JP)
(72) Erfinder: Okuda, Kazuhiko, c/o Keisokki Kogyo Co., Ltd., Hyogo-ken, 661-0021 (JP); Stein, Wolfgang, Dr., 41239 Mönchengladbach (DE)
(74) Vertreter: Dallmeyer, Georg, Dipl.-Ing.

(56) Entgegenhaltungen:
- EP-A- 0 924 518
- DE-A- 3 621 324
- US-A- 3 069 621
- US-A- 5 442 447
- US-A- 5 530 368

## Beschreibung

Die Erfindung betrifft eine Vorrichtung zum automatischen Prüfen der Gleichmäßigkeit von textilem Prüfgut, insbesondere von Garnen, Bändern und Vorgarnen nach dem Oberbegriff des Anspruchs 1.

Eine derartige Vorrichtung dient dazu, qualitätsbeschreibende und qualitätsbestimmende Kenngrößen an linienförmigen textilen Materialien automatisch bei On-line-Erfassung der Meßdaten zu bestimmen. Unterschiedliche Prüfgutsorten können automatisch nacheinander gemessen werden.

Die Messung und Überwachung dieser Kenngrößen erfolgt in einem Meßspalt eines kapazitiven Meßorgans.

Eine für die Weiterverarbeitung von linienförmigen Textilien wichtige und qualitätsbestimmende Kenngröße in Spinnereien ist die Ungleichmäßigkeit der Masseverteilung über der Prüfgutlänge. Sie charakterisiert die Abweichung der realen Masseverteilung von einer homogenen theoretischen Verteilung über die Prüfgutlänge.

Bekannte Gleichmäßigkeitsprüfer sind so aufgebaut, daß der kapazitive Messkopf aus einer festen Anzahl von Messspalten mit unterschiedlicher Spaltweite besteht, wobei jeder Messspalt für einen bestimmten Feinheitsbereich des Prüfgutes verwendet wird. Ein derartiger Gleichmäßigkeitsprüfer ist z.B. aus der EP-A-0 266 611 bekannt. Hierbei werden die unterschiedlichen Dielektrizitätskonstanten des Fasermaterials, aus dem das Prüfgut besteht, nicht berücksichtigt. Die Anpassung des Messsignals beim Durchlauf des Prüfgutes durch den Messspalt bei unterschiedlicher Dielektrizitätskonstante des Faserverbandes wird durch eine unterschiedliche elektronische Verstärkung des Messsignals erreicht.

Die Verwendung von mehreren Messspalten für unterschiedliche Feinheiten der Proben, sowie des gleichen Messspaltes für gleiche Feinheiten verschiedener Faserqualitäten mit unterschiedlicher Dielektrizitätskonstante stellt bei den bisher bekannten Gleichmäßigkeitsprüfern eine Ursache für eine nicht zufriedenstellende Reproduzierbarkeit der Messergebnisse am gleichen Prüfgut dar.

Ein gattungsgemäßer Stand der Technik ist aus der DE 36 21 324 A bekannt. Bei dem bekannten Stand der Technik sind zentrale feststehende Elektroden vorgesehen, wobei jede der feststehenden Elektroden einer beweglichen Elektrode gegenübersteht, die von einem Schrittmotor derart bewegt werden kann, dass die wirksame Breite des Messspaltes an der Stelle durchlaufenden Prüfgutes variabel einstellbar ist. Dabei wird der Messspalt der beiden Kondensatoren gleichzeitig verändert.

Der Erfindung liegt die Aufgabe zugrunde, einen Gleichmäßigkeitsprüfer der eingangs genannten Art zu schaffen, der hinsichtlich der Positionierung des Prüfgutes sowie bezüglich der Messgenauigkeit und der Reproduzierbarkeit der Messwerte verbessert ist.

Zur Lösung dieser Aufgabe dienen die Merkmale des Anspruchs 1.

Die Erfindung sieht in vorteilhafter Weise vor, daß die für die Messung wirksame Breite des Messspaltes an der Stelle des durchlaufenden Prüfgutes mit Hilfe einer Positioniereinrichtung bei in dem Messspalt verbleibendem Prüfgut kontinuierlich oder in Stufen einstellbar ist, wobei der Messspalt nach außen divergiert und die Positioniereinrichtung den Messkopf oder das Prüfgut derart zueinander verschiebt, dass die für die Messung wirksame Breite veränderbar ist.

Ein derartiger Messkopf weist nur noch einen einzigen Messspalt auf, in dem Prüfgut unterschiedlicher Feinheit gemessen werden kann. Mit Hilfe der Positioniereinrichtung kann die relative Lage des Prüfgutes im Messkopf und damit die wirksame Breite des Messspaltes eingestellt werden, wodurch in einfacher Weise eine Vielzahl von Meßspaltbreiten für eine Messung zur Verfügung stehen.

Vorzugsweise bestehen die den Meßkondensator und den Referenzkondensator bildenden Kondensator-Elektroden aus Kondensatorplatten.

Bei einem besonders bevorzugten Ausführungsbeispiel ist vorgesehen, daß mindestens eine Kondensator-Elektrode eine von der Befestigungsplatte ausgehend schräg verlaufende, dem Meßspalt zugewandte Oberfläche aufweist, wodurch die Breite des Meßspaltes mit zunehmenden Abstand von der Befestigungsplatte zunimmt. Durch die in bezug auf eine schräg zum Prüfgut verlaufende Oberfläche mindestens einer der Kondensator-Elektroden wird ein relativ zur Befestigungsplatte nach außen divergierender Meßspalt gebildet. Je nach Positionierung des Prüfgutes innerhalb des Meßspaltes steht eine unterschiedliche wirksame Meßspaltbreite durch Einstellung der Durchlaufposition des Prüfgutes oder der Meßkopfposition zur Verfügung.

Alternativ kann vorgesehen sein, daß mindestens eine der einander zugewandten Kondensatoroberflächen eine stufenförmige Oberfläche aufweist, wobei die Stufen parallel zu dem Prüfgut verlaufen. Auch bei diesem Ausführungsbeispiel kann durch die Positionierung des Prüfgutes relativ zu dem Meßkopf die wirksame Breite des Meßspaltes eingestellt werden.

Bei einem bevorzugten Ausführungsbeispiel ist vorgesehen, daß die Positioniereinrichtung den Meßkopf relativ zu dem Prüfgut in Richtung zu- oder abnehmender Breite des Meßspaltes verschiebt.

Dadurch kann bei unveränderter Durchlaufposition des Prüfgutes der Meßkopf in Richtung zu- oder abnehmender Breite des Meßspaltes relativ zu dem Prüfgut verschoben werden und somit die wirksame Meßspaltbreite eingestellt werden.

Das Prüfgut kann alternativ durch Verschieben von Führungseinrichtungen innerhalb des Meßspaltes positioniert werden.

Es ist dabei vorgesehen, daß die Positioniereinrichtung Führungseinrichtungen mit dem Prüfgut relativ zu dem Meßkopf in Richtung zu- oder abnehmender Breite des Meßspaltes verschiebt. Dadurch wird das Prüfgut innerhalb des Meßspaltes parallel vorund zurückpositioniert, wodurch die wirksame Breite des Meßspaltes eingestellt wird. Zusätzlich kann die Positioniereinrichtung die Führungseinrichtungen auch seitlich verschieben, so daß der Abstand des Prüfgutes von den Kondensator-Elektroden des Meßkondensators verändert werden kann. Dabei kann insbesondere ein unsymmetrischer Abstand von den den Meßspalt begrenzenden Kondensator-Elektroden eingestellt werden.

Zusätzlich kann auch mindestens eine der Kondensator-Elektroden quer zur Laufrichtung des Prüfgutes in Richtung zu- oder abnehmender Breite des Meßspaltes auf der Befestigungsplatte parallel verschoben werden. Auch in diesem Fall kann die Durchlaufposition des Prüfgutes unverändert bleiben.

Bei allen alternativen Positioniereinrichtungen ist der Antrieb mit einer Wegmeßeinrichtung gekoppelt, die ein Meßsignal an die Steuerung überträgt, die auf diese Weise die effektive Meßspaltbreite berechnen kann.

Das Prüfgut kann an mindestens einer Kondensator-Elektrode anliegen. Dabei liegt das Prüfgut vorzugsweise an der inneren Kondensatorplatte des Meßspaltes an, wodurch ein unruhiger Lauf des Prüfgutes während der Messung verhindert wird.

Ein bevorzugte Weiterbildung der Erfindung sieht vor, daß die Steuerung den Antrieb für eine Verschiebung der Kondensator-Elektroden und/oder der Führungseinrichtung für das Prüfgut ansteuert, bis das Meßsignal des Meßkondensators mit durchlaufendem oder stillstehendem Prüfgut eine vorgegebene Meßsignalgröße erreicht. Auf diese Weise kann durch Eingabe der Feinheit des Prüfgutes und einer sogenannten Materialkennzahl, welche den Einfluß der dieelektrischen Eigenschaften des Faserstoffs beschreibt, der Meßspalt so eingestellt werden, daß eine vorbestimmte Meßsignalgröße erreicht wird. Dadurch kann die Meßspaltbreite in bezug auf das Prüfgut optimiert werden, wodurch die Meßsignalstärke verbessert und Meßwertungenauigkeiten reduziert werden sowie die Reproduzierbarkeit der Meßwerte erhöht wird. Die Materialkennziffer ist dabei eine abgeleitete Größe, die auf den Dielektrizitätskonstanten der unterschiedlichen Faserstoffe beruht.

Die Kondensator-Elektroden können eine isolierende Beschichtung aufweisen.

Der Meßkopf kann für eine zwischenzeitliche Kalibrierung parallel zum Meßspalt und orthogonal zur Befestigungsplatte so weit zurückgezogen werden, bis sich das Prüfgut außerhalb des Meßspaltes befindet. Dabei wird der Meßkopf in das Gehäuse der Vorrichtung zurückgezogen, bis das Prüfgut freiliegt.

Desweiteren kann vorgesehen sein, daß mindestens eine der Kondensator-Elektroden austauschbar ist. Vorzugsweise ist die mittlere Kondensatorplatte austauschbar. Die Befestigungsplatte weist hierzu beispielsweise eine Steckverbindungseinrichtung auf, wobei diese ein Kodiersignal an die Steuerung übertragen kann. Dadurch kann die Steuerung die eingesetzte Kondensatorplattengröße erkennen.

Im Folgenden werden unter Bezugnahme auf die Zeichnungen Ausführungsbeispiele der Erfindung näher erläutert:

Es zeigen:
- Fig. 1: ein Gleichmäßigkeitsprüfgerät mit einem erfindungsgemäßen Meßkopf,
- Fig. 2: eine Draufsicht auf ein erstes Ausführungsbeispiel eines Meßkopfes,
- Fig. 3: eine Draufsicht auf ein zweites Ausführungsbeispiel eines Meßkopfes,
- Fig. 4: eine Seitenansicht des Meßkopfes gemäß Fig. 3 ohne Führungseinrichtung in zurückgezogener Position,
- Fig. 5: eine Seitenansicht einer Führungseinrichtung für das Prüfgut am Meßkopf,
- Fig. 6: eine Frontansicht der Führungseinrichtung am Meßkopf,
- Fig. 7: ein drittes Ausführungsbeispiel eines Meßkopfes, und
- Fig. 8: eine Frontansicht des Meßkopfes gemäß Fig. 7.

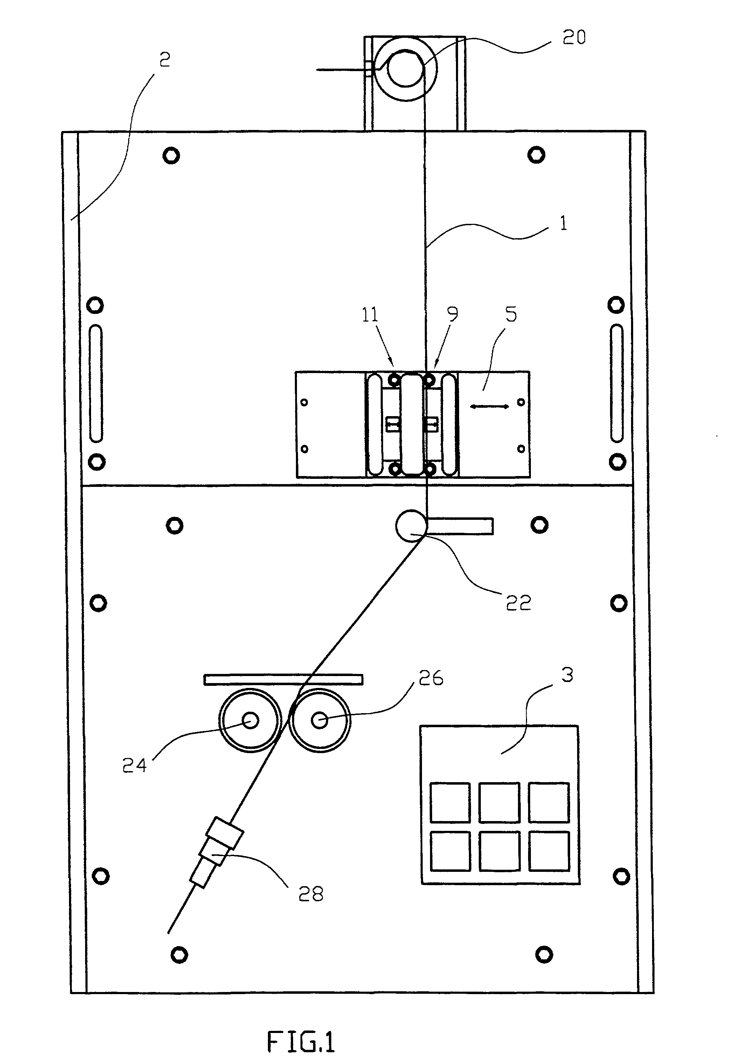

Fig. 1 zeigt einen Gleichmäßigkeitsprüfer mit einem Gehäuse 2 und einem kapazitiven Meßkopf 5 mit einem Meßkondensator 9 und einem Referenzkondensator 11. Das Prüfgut 1 besteht aus linienförmigen textilen Materialien, insbesondere aus Garnen, Bändern und Vorgarnen. Ein derartiges Gleichmäßigkeitsprüfgerät ermöglicht die automatische On-line-Erfassung der Ungleichmäßigkeit der Massenverteilung über die Prüfgutlänge. Dabei werden unterschiedlich statistische Daten erfaßt und berechnet.

Eine Steuerung 3 koordiniert den Transport des Prüfgutes 1, die Durchführung und Auswertung der Messung und die Einstellungen an dem Meßkopf 5 mit Hilfe von Positioniereinrichtungen 44,46.

Der Meßkopf 5 weist mehrere zueinander parallele Kondensator-Elektroden 10,12,14 sowie zwei äußere Abschirm-Elektroden 17 auf, die orthogonal von einer Befestigungsplatte 6 abstehen. Die mittlere Kondensator-Elektrode 12 sowie die Abschirm-Elektroden 17 liegen an Masse an, während die Kondensator-Elektroden 10,14 auf Potential liegen. Die mittlere Kondensator-Elektrode 12 bildet in Verbindung mit der einen Kondensator-Elektrode 14 und dem dazwischen liegenden Meßspalt 8 den Meßkondensator 9. Die gleiche mittlere Kondensator-Elektrode 12 bildet desweiteren in Verbindung mit der Kondensator-Elektrode 10 und dem dazwischen liegenden Referenzmeßspalt 7 den Referenzkondensator 11. Es versteht sich, daß der Meßkondensator 9 auch als Referenzkondensator und der Referenzkondensator 11 auch als Meßkondensator verwendet werden können.

Das textile Prüfgut 1 wird bei einem Gleichmäßigkeitsprüfgerät, wie in Fig. 1 gezeigt, beispielsweise über eine Tellerbremse 20 vertikal durch den Meßspalt 8 und unterhalb des Meßkopfes 5 über eine Umlenkeinrichtung 22 zwischen zwei Walzen 24,26 einer Absaugeinrichtung 28 zugeführt, so daß eine kontinuierliche Messung an dem Prüfgut 1 möglich ist.

Die Kondensator-Elektroden 10,12,14 sind auf der Befestigungsplatte 6 einseitig befestigt montiert, wobei zumindest die mittlere Kondensator-Elektrode 12 austauschbar ist. Der Austausch der mittleren Kondensator-Elektrode 12 erfolgt z.B. über eine Steckverbindung. Der Steckverbinder kann kodiert sein, so daß die Steuerung 3 ein Signal erhält, welche Kondensator-Elektrode 12 eingesetzt ist. Dadurch können Kondensator-Elektroden 12 mit Meßspalten 8,9 unterschiedlicher Breite eingesetzt werden, um eine Grobanpassung an sehr unterschiedliche Materialdichten zu erreichen.

Selbstverständlich ist auch der gesamte Meßkopf austauschbar.

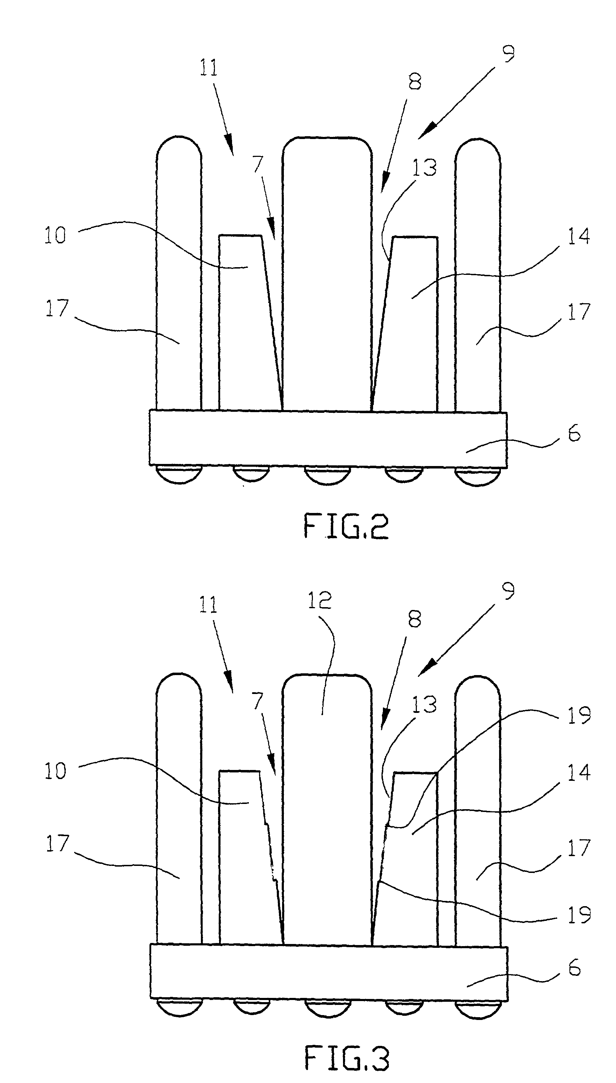

Gemäß Fig. 2 ist vorgesehen, daß der Meßspalt 8 nach außen divergiert. Hierzu ist eine der beiden Kondensator-Elektroden 12,14, vorzugsweise die auf Potential liegende Kondensator-Elektrode 14, mit einer in Draufsicht gemäß Fig. 2 von der Befestigungsplatte 6 ausgehend schräg verlaufenden, dem Meßspalt 8 zugewandten Seitenfläche 13 versehen. Dadurch nimmt die Breite des Meßspaltes 8 mit zunehmenden Abstand von der Befestigungsplatte 6 zu.

Vorzugsweise ist vorgesehen, daß der Referenzkondensator 11 spiegelsymmetrisch gestaltet ist und somit ebenfalls einen nach außen divergierenden Meßspalt als Referenzmeßspalt 7 aufweist. In Fig. 2 sind die Abschirm-Elektroden 17 und die mittlere Kondensator-Elektrode 12 mit zueinander parallelen Seitenflächen versehen. Alternativ kann die mittlere Kondensator-Elektrode 12 sich nach außen konisch verjüngen, wobei die Kondensator-Elektroden 10 und 14 dann zueinander parallele Seitenflächen 13 aufweisen.

Fig. 3 zeigt ein alternatives Ausführungsbeispiel, bei dem die dem Meßspalt 8 bzw. dem Referenzmeßspalt 7 zugewandten Seitenflächen der auf Potential liegenden Kondensator-Elektroden 10,14 eine gestufte Seitenfläche 13 aufweisen, wobei die Stufenkanten 19 parallel zu dem Prüfgut 1 verlaufen. Bei dem Ausführungsbeispiel der Fig. 3 kann das Prüfgut 1 an drei unterschiedlichen Stellen durch den Meßkopf 5 mit unterschiedlicher wirksamer Breite des Meßspaltes 8 hindurchlaufen. Zusätzlich können auch die gestufte Seitenfläche schräg verlaufen, wie in Fig. 3 dargestellt. Vorzugsweise haben alle drei Stufen in Fig. 3 die gleiche Schräge.

Auch bei diesem Aufführungsbeispiel ist der Referenzkondensator 11 vorzugsweise spiegelbildlich zu dem Meßkondensator 9 gestaltet.

Das Prüfgut 1 wird mit Hilfe einer Positioniereinrichtung 44,46 relativ zu dem Meßkopf 5 in dem Meßspalt 8 positioniert, so daß mit Hilfe der Positioniereinrichtung 44,46 die wirksame Breite des Meßspaltes 8 wählbar ist. Die Positioniereinrichtung 44,46 erlaubt demzufolge die relative Positionierung des Prüfgutes 1 in dem Meßspalt 8 in bezug auf die Tiefe des Meßspaltes.

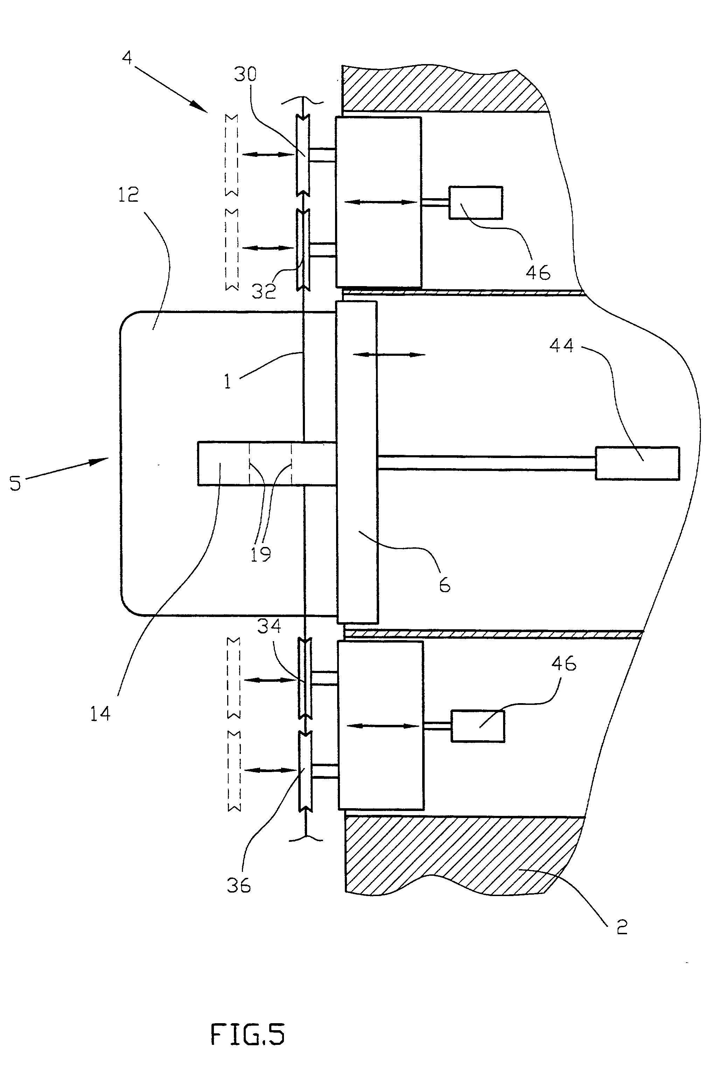

Wie am besten aus Fig. 5 ersichtlich ist, bestehen zwei Möglichkeiten, die relative Position des Prüfgutes 1 im Meßspalt 8 einzustellen. Wenn eine Führungseinrichtung 4 vorhanden ist, kann das Prüfgut 1 mit einer Führungseinrichtung 4 relativ zum dem Meßkopf 5 und dem Gehäuse 2 mit Hilfe der Positioniereinrichtung 46 vor- und zurückbewegt werden, so daß bei einem sich stufenförmig oder kontinuierlich erweiternden Meßspalt 8 unterschiedliche Durchlaufpositionen für das Prüfgut 1 mit Hilfe der Steuerung 3 angesteuert werden können. Falls keine Führungseinrichtung 4 vorgesehen ist, kann der Meßkopf 5 mit Hilfe einer Positioniereinrichtung 44 relativ zu dem Gehäuse vor- und zurückbewegt werden, wodurch ebenfalls bei einem sich stufenförmig oder kontinuierlich erweiternden Meßspalt 8 unterschiedliche wirksame Meßspaltbreiten eingestellt werden können. Die Ausführungsform, bei der mit Hilfe der Positioniereinrichtung 44 die wirksame Meßspaltbreite eingestellt wird, stellt die bevorzugte Ausführungsform dar, da in diesem Fall die Führungseinrichtung 4 vollständig entfallen kann.

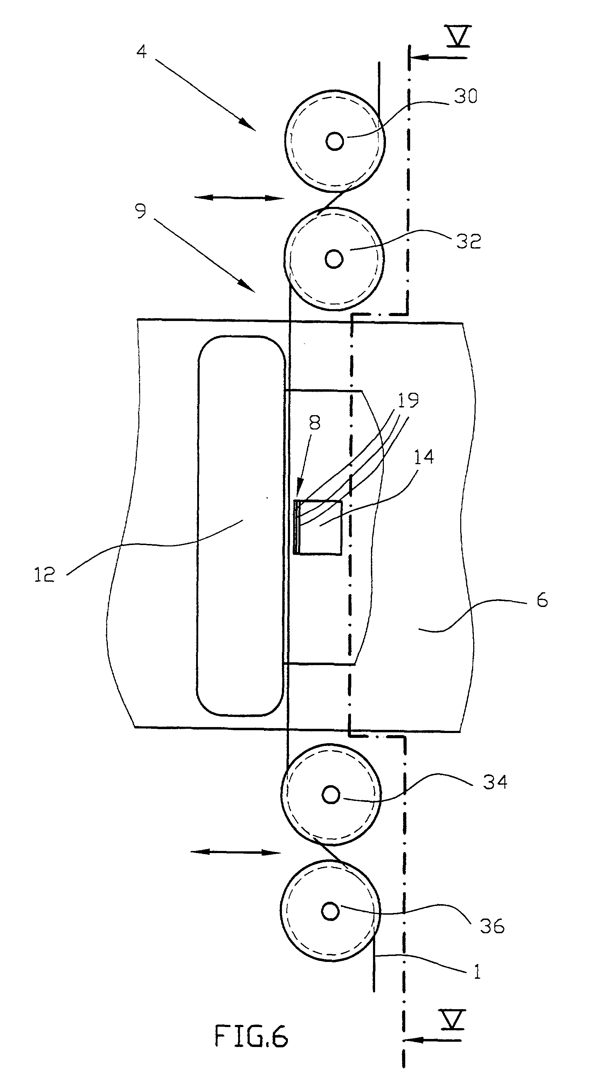

Zusätzlich kann die Führungseinrichtung 4 dazu verwendet werden, das Prüfgut 1 auch geringfügig seitlich, wie aus Fig. 6 ersichtlich, zu positionieren, d.h. in Richtung auf die Kondensator-Elektrode 12 oder 14.

Die Führungseinrichtung 4 besteht aus oberhalb und unterhalb des Meßkopfes 5 angeordneten keramischen Umlenkkörpern 30,32 sowie 34,36, die auch aus Umlenkrollen bestehen können.

Die Umlenkkörper 30 bis 36 sind mit einer Führungsrille 40 versehen, in der das Prüfgut 1 umgelenkt wird.

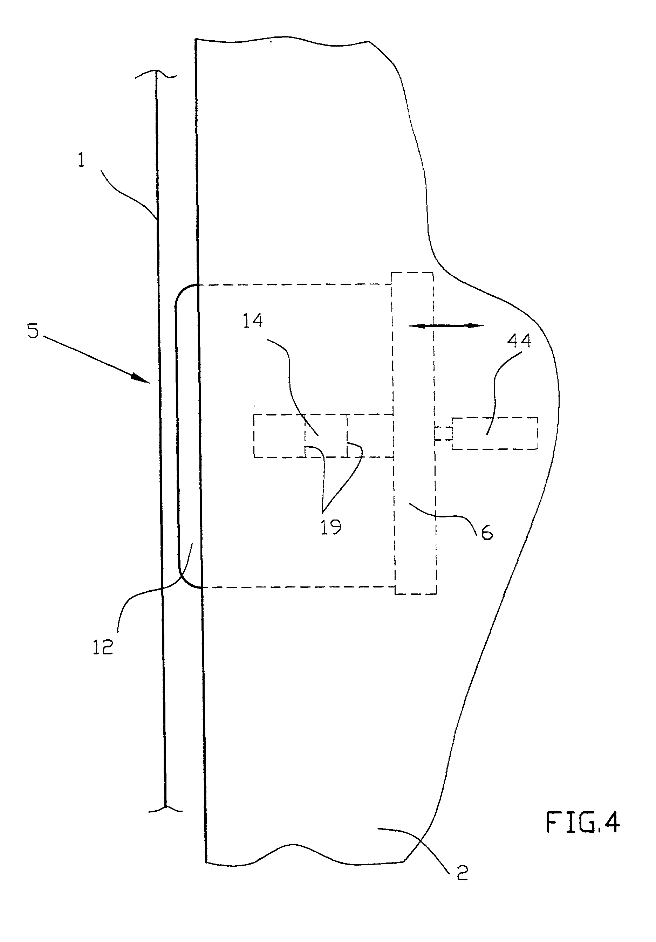

Das Prüfgut wird von der Tellerbremse 20 vertikal und im wesentlichen parallel zur Frontfläche des Gehäuses 2 dem oberen Umlenkkörper 30 zugeführt, von diesem umgelenkt und anschließend nach weiterer Umlenkung durch den Umlenkkörper 32 dem Meßspalt 8 vertikal zugeführt. Unterhalb des Meßkopfes wird das Prüfgut 1 wiederum zweimal von den unteren Umlenkkörpern 34,36, wie in Fig. 6 ersichtlich, umgelenkt. Zwischen dem Umlenkkörper 32 und dem Umlenkkörper 34 ist das Prüfgut 1 exakt parallel zu den plattenförmigen Kondensator-Elektroden 12,14 geführt. Die Einstellung mit Hilfe der Führungseinrichtung 4 kann auch so erfolgen, daß das Prüfgut 1 an einer der beiden Kondensatorelektroden 12,14, vorzugsweise an der mittleren Kondensator-Elektrode 12, anliegt. Die Kondensator-Elektrode 14 in den Fign. 4, 5 und 6 entspricht dem Ausführungsbeispiel der Fig. 3.

Die Oberflächen der Kondensator-Elektroden 10,12,14 können mit einer Isolierschicht versehen sein.

Bei dem zuvor erläuterten bevorzugten Ausführungsbeispiel, wie in Fig. 4 dargestellt, ist vorgesehen, daß die Positioniereinrichtung 44 den Meßkopf 5 relativ zu dem Prüfgut 1 in Richtung zu- oder abnehmender Breite des Meßspaltes 8, d.h. relativ zu dem Gehäuse 2 vor und zurück verschiebt. In diesem Fall ist keine zusätzliche Führungseinrichtung 4 erforderlich. Die Positioniereinrichtung 44 besteht beispielsweise aus einer Motor/Spindel-einheit oder einer Kolben-Zylindereinheit, die einerseits mit der Befestigungsplatte 6 und andererseits mit dem Gehäuse 2 gekoppelt ist.

Die gleiche Positioniereinrichtung 44 kann bei allen Ausführungsbeispielen auch dazu verwendet werden, den Meßkopf 5 für eine zwischenzeitliche Kalibrierung soweit parallel nach innen in das Gehäuse 2 zu verschieben, daß sich das Prüfgut 1 außerhalb des Meßspaltes 8 befindet.

Alle Positioniereinrichtungen 44,46 nach den Ausführungsbeispielen der Fign. 4 bis 6 haben gemeinsam, daß sie mit einer in den Zeichnungen nicht dargestellten Wegmeßeinrichtung gekoppelt sind, die ein Meßsignal an die Steuerung 3 überträgt, aus der die Steuerung 3 die wirksame Breite des Meßspaltes 8 ermittelt.

Die Steuerung 3 kann auch die Positioniereinrichtungen 44,46 zur Einstellung der wirksamen Breite des Meßspaltes 8 ansteuern, bis das Meßsignal des Meßkondensators 9 bei durchlaufendem oder stillstehendem Prüfgut 1 eine vorgegebene Meßsignalgröße erreicht.

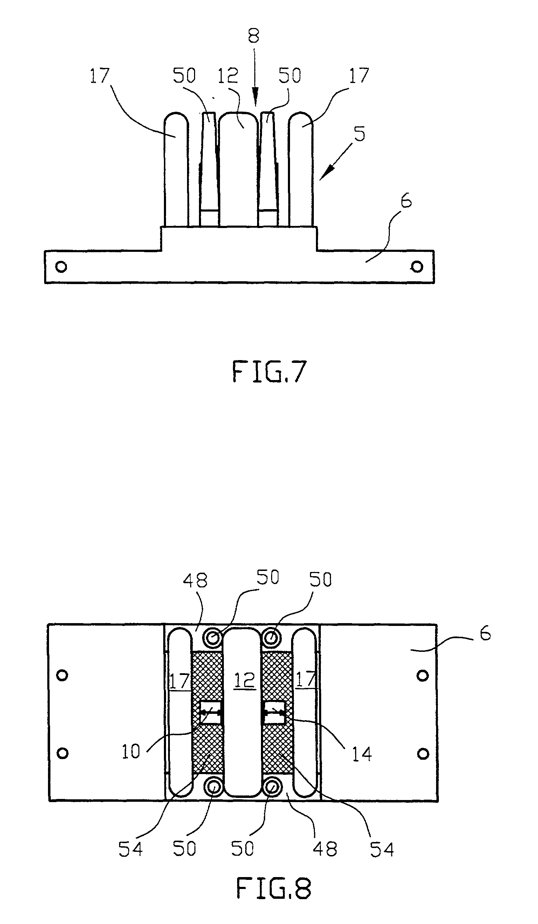

Bei dem Ausführungsbeispiel der Fign. 7 und 8 ist ein Meßkopf 5 dargestellt, der zusätzlich mit konischen Führungsstiften 50 versehen ist, die das Einfädeln des Prüfgutes 1 in den Meßspalt 8 erleichtern.

Die Befestigungsplatte 6 aus Metall besteht wie bei allen Ausführungsbeispielen aus zwei Teilen, die mit metallischen Stegen 48 miteinander verbunden sind. Die Abschirmelektroden 17 sowie die mittlere Kondensator-Elektrode 12 sind auf den mit Masse verbundenen Stegen 48 montiert. Dagegen sind die auf Potential liegenden Kondensator-Elektroden 10,14 auf einer Isolationsplatte 54 montiert, die zwischen den Stegen 48 und den beiden Teilen der Befestigungplatte 6 angeordnet ist.

Wie aus Fig. 8 ersichtlich sind die Kondensator-Elektroden 10,14 zur Veränderung der Breite des Meßspaltes 8 und des Referenzmeßspaltes 7 mit Hilfe einer nicht dargestellten Positioniereinrichtung orthogonal zu den Abschirmplatten 17 und der mittleren Kondensator-Elektrode 12 in Richtung zu- oder abnehmender Breite des Meßspaltes 8 bzw. des Referenzmeßspaltes 7 seitlich verschiebbar.

Zusätzlich kann der gesamte Meßkopf 5 für eine zwischenzeitliche Kalibrierung in das Gehäuse 2 zurückgezogen werden, wie bereits in Verbindung mit anderen Ausführungsbeispielen erläutert.

## Patentansprüche

1. Vorrichtung zum automatischen Prüfen der Gleichmäßigkeit von textilem Prüfgut (1), insbesondere von Garnen, Bändern und Vorgarnen,
- mit einem Gehäuse (2), an dem das textile Prüfgut (1) kontinuierlich durch einen aus mehreren auf einer Befestigungsplatte (6) einseitig befestigten Kondensator-Elektroden (10,12,14,17) gebildeten Messkopf (5) hindurchgeführt wird, wobei die Kondensator-Elektroden (10,12,14, 17) in Richtung des Prüfgutes (1) parallel zueinander nebeneinander angeordnet sind,
- wobei das Prüfgut (1) zwischen den Kondensator-Elektroden (12,14) eines Messkondensators (9) in einen Messspalt (8) geführt ist, und
- wobei die für die Messung wirksame Breite des Messspaltes (8) an der Stelle des durchlaufenden Prüfgutes (1) mit Hilfe einer Positioniereinrichtung (44,46) bei in dem Messspalt (8;9) verbleibendem Prüfgut (1) einstellbar ist, und
- mit einer Steuerung (3) für den Transport des Prüfgutes (1), die Durchführung der Messung und die Auswertung des Messsignals,
**dadurch gekennzeichnet,**
**dass** der Messspalt (8) nach außen divergiert und dass die Positioniereinrichtung (44,46) den Messkopf (5) oder das Prüfgut (1) derart zueinander verschiebt, dass die für die Messung wirksame Breite veränderbar ist.

2. Vorrichtung nach Anspruch 1, **dadurch gekennzeichnet, daß** die Kondensator-Elektroden (10,12,14) aus Kondensatorplatten bestehen.

3. Vorrichtung nach Anspruch 1 oder 2, **dadurch gekennzeichnet, daß** mindestens eine Kondensator-Elektrode (10,12,14) eine in einer Querschnittsebene durch das Prüfgut (1) schräg verlaufende, dem Meßspalt (8) zugewandte Seitenfläche (13) aufweist, wodurch die Breite des Meßspaltes (8) mit zunehmendem Abstand von der Befestigungsplatte (6) zunimmt.

4. Vorrichtung nach Anspruch 1 bis 3, **dadurch gekennzeichnet, daß** mindestens eine der einander zugewandten seitlichen Kondensatoroberflächen (13) eine dem Meßspalt (7,8) zugewandte gestufte Seitenfläche (13) aufweist, wobei die Stufenkanten (19) parallel zu dem Prüfgut (1) verlaufen.

5. Vorrichtung nach einem der Ansprüche 1 bis 4, **dadurch gekennzeichnet, daß** die Positioniereinrichtung (44) den Meßkopf (5) relativ zu dem Prüfgut (1) in Richtung zu- oder abnehmender Breite des Meßspaltes (8) verschiebt.

6. Vorrichtung nach einem der Ansprüche 1 bis 4, **dadurch gekennzeichnet, daß** die Positioniereinrichtung (46) eine Führungseinrichtung (4) für das Prüfgut (1) relativ zu dem Meßkopf (5) in Richtung zu- oder abnehmender Breite des Meßspaltes (8) verschiebt.

7. Vorrichtung nach einem der Ansprüche 1 bis 4, **dadurch gekennzeichnet, daß** Positioniereinrichtung mindestens eine Kondensator-Elektrode (10,14) quer zur Laufrichtung des Prüfgutes (1) in Richtung zu- oder abnehmender Breite des Meßspaltes (8) parallel verschiebt.

8. Vorrichtung nach einem der Ansprüche 5 bis 7, **dadurch gekennzeichnet, daß** der Antrieb der Positioniereinrichtung (44,46) mit einer Wegmeßeinrichtung gekoppelt ist, die ein Meßsignal an die Steuerung (3) überträgt.

9. Vorrichtung nach einem der Ansprüche 1 bis 7, **dadurch gekennzeichnet, daß** das Prüfgut (1) an mindestens einer Kondensator-Elektrode (12,14) anliegt.

10. Vorrichtung nach einem der Ansprüche 5 bis 9, **dadurch gekennzeichnet, daß** die Steuerung (3) die Positioniereinrichtung (44,46) zur Einstellung der effektiven Breite des Meßspaltes (8) ansteuert, bis das Meßsignal des Meßkondensators (9) bei durchlaufendem oder stillstehendem Prüfgut (1) eine vorgegebene Größe erreicht.

11. Vorrichtung nach einem der Ansprüche 1 bis 10, **dadurch gekennzeichnet, daß** mindestens eine der Kondensator-Elektroden - (10,12,14) eine isolierende Beschichtung aufweist.

12. Vorrichtung nach einem der Ansprüche 1 bis 11, **dadurch gekennzeichnet, daß** der Meßkopf (5) für eine zwischenzeitliche Kalibrierung parallel zum Meßspalt (7,8) und orthogonal zur Befestigungsplatte (6) so weit verschiebbar ist, bis sich das Prüfgut (1) außerhalb des Meßspaltes (8) befindet.

13. Vorrichtung nach einem der Ansprüche 1 bis 12, **dadurch gekennzeichnet, daß** mindestens eine der Kondensator-Elektroden (10,12,14) austauschbar ist.

## Claims

1. A device for automatically testing the uniformity of a textile test specimen (1), in particular yarns, bands, and roving yarns,
- comprising a housing (2) at which the textile test specimen (1) is continuously passed through a measuring head (5) formed by a plurality of capacitor electrodes (10, 12, 14, 17) having one side fixed to a mounting plate (6), the capacitor electrodes (10, 12, 14, 17) being arranged side by side in parallel in the direction of the test specimen (1),
- the test specimen (1) being guided into a measuring gap (8) between the capacitor electrodes (12, 14) of a measuring capacitor (9), and
- the width of the measuring gap (8) effective for the measurement is adjustable at the position of the passing test specimen (1) using a positioning device (44, 46), with the test specimen (1) remaining in the measuring gap (8; 9), and
- comprising a control (3) for transporting the test specimen (1) and for performing the measurement and the evaluation of the measuring signal,
**characterized in that**
- the measuring gap (8) diverges outward and the positioning means (44, 46) displaces the measuring head (5) or the test specimen (1) relative to each other such that the width effective for measuring is adjustable.

2. The device of claim 1, wherein the capacitor electrodes (10, 12, 14) are capacitor plates.

3. The device of claim 1 or 2, wherein at least one capacitor electrode (10, 12, 14) has a side surface (13) extending obliquely in a cross-sectional plane through the test specimen (1) and facing the measuring gap (8), the width of the measuring gap (8) increasing with the distance from the mounting plate (6).

4. The device of claims 1 to 3, wherein at least one of the facing capacitor side surfaces (13) has a stepped side surface (13) facing the measuring gap (7, 8), the edges (19) of the steps extending in parallel with the test specimen (1).

5. The device of one of claims 1 to 4, wherein the positioning device (44) displaces the measuring head (5) relative to the test specimen (1) in the increasing or decreasing width direction of the measuring gap (8).

6. The device of one of claims 1 to 4, wherein the positioning device (46) displaces a guide means (4) for the test specimen (1) relative to the measuring head (5) in the increasing or decreasing width direction of the measuring gap (8).

7. The device of one of claims 1 to 4, wherein the positioning device displaces at least one capacitor electrode (10, 14) transversely to the running direction of to the test specimen (1) in the increasing or decreasing width direction of the measuring gap (8).

8. The device of one of claims 5 to 7, wherein the drive of the positioning device (44, 46) is coupled to a path sensor sending a measuring signal to the control (3).

9. The device of one of claims 1 to 7, wherein the test specimen (1) contacts at least one capacitor electrode (12, 14).

10. The device of one of claims 5 to 9, wherein the control (3) drives the positioning device (44, 46) for adjusting the effective width of the measuring gap (8) until the measuring signal of the measuring capacitor (9) reaches a predetermined value, with the test specimen (1) running or at standstill.

11. The device of one of claims 1 to 10, wherein at least one of the capacitor electrodes (10, 12, 14) is provided with an insulating coating.

12. The device of one of claims 1 to 11, wherein, for intermittent calibration, the measuring head (5) may be displaced in parallel to the measuring gap (7, 8) and orthogonal to the mounting plate (6) until the test specimen (1) is located without the measuring gap (8).

13. The device of one of claims 1 to 12, wherein at least one of the capacitor electrodes (10, 12, 14) is replaceable.

## Revendications

1. Appareil pour tester automatiquement la régularité d'un produit textile à vérifier (1), en particulier fils, bandes et mèches, comportant
- un boîtier (2) dans lequel le produit textile à vérifier (1) traverse en continu une tête de mesure (5) formée de plusieurs électrodes-condensateurs (10, 12, 14) fixées d'un côté à une plaque de fixation (6), les électrodes-condensateurs (10, 12, 14) étant disposées en parallèle les unes à côté des autres suivant la direction du produit à vérifier (1),
- le produit à vérifier (1) étant guidé dans une fente de mesure (8) entre les électrodes-condensateurs (12, 14) d'un condensateur de mesure (9), et
- la largeur efficace pour la mesure, de la fente de mesure (8), étant réglable à l'endroit où passe le produit à vérifier (1) à l'aide d'un dispositif de positionnement (44, 46) lorsque le produit à vérifier (1) demeure dans la fente de mesure (8 ; 9), et
- comportant une commande (3) pour l'avance du produit à vérifier (1), la mise en oeuvre de la mesure et l'exploitation du signal de mesure,
**caractérisé en ce que**
la fente de mesure (8) diverge vers l'extérieur et **en ce que** le dispositif de positionnement (44, 46) déplace la tête de mesure (5) ou le produit à vérifier (1) de telle sorte que la largeur efficace pour la mesure puisse être modifiée.

2. Appareil selon la revendication 1, **caractérisé en ce que** les électrodes-condensateurs (10, 12, 14) sont constituées par des plaques de condensateur.

3. Appareil selon la revendication 1 ou 2, **caractérisé en ce qu'** au moins une électrode-condensateur (10, 12, 14) présente une face latérale (13) tournée vers la fente de mesure (8) et s'étendant en oblique dans un plan de section transversale du produit à vérifier (1), de sorte que la largeur de la fente de mesure (8) augmente à mesure que la distance à la plaque de fixation (6) augmente.

4. Appareil selon revendication 1 à 3, **caractérisé en ce qu'**au moins l'une des surfaces de condensateur (13) latérales tournées l'une vers l'autre comporte une face latérale (13) en gradins tournée vers la fente de mesure (7, 8), les bords des gradins (19) s'étendant parallèlement au produit à vérifier (1).

5. Appareil selon l'une des revendications 1 à 4, **caractérisé en ce que** le dispositif de positionnement (44) déplace la tête de mesure (5) par rapport au produit à vérifier (1) dans le sens de l'augmentation ou de la diminution de la largeur de la fente de mesure (8).

6. Appareil selon l'une des revendications 1 à 4, **caractérisé en ce que** le dispositif de positionnement (46) déplace un dispositif (4) de guidage du produit à vérifier par rapport à la tête de mesure (5) dans le sens de l'augmentation ou de la diminution de la largeur de la fente de mesure (8).

7. Appareil selon l'une des revendications 1 à 4, **caractérisé en ce que** le dispositif de positionnement déplace en parallèle au moins une électrode-condensateur (10, 14) transversalement à la direction de défilement du produit à vérifier (1) dans le sens de l'augmentation ou de la diminution de la largeur de la fente de mesure (8).

8. Appareil selon l'une des revendications 5 à 7, **caractérisé en ce que** l'entraînement du dispositif de positionnement (44, 46) est couplé à un dispositif de mesure de déplacement qui transmet un signal de mesure à la commande (3).

9. Appareil selon l'une des revendications 1 à 7, **caractérisé en ce que** le produit à vérifier (1) est adjacent à au moins une électrode-condensateur (12, 14).

10. Appareil selon l'une des revendications 5 à 9, **caractérisé en ce que** la commande (3) agit sur le dispositif de positionnement (44, 46) en vue de régler la largeur utile de la fente de mesure (8) jusqu'à ce que le signal de mesure du condensateur de mesure (9) atteigne une grandeur prédéterminée lorsque le produit à vérifier (1) est en mouvement ou bien au repos.

11. Appareil selon l'une des revendications 1 à 10, **caractérisé en ce qu'** au moins l'une des électrodes-condensateurs (10, 12, 14) comporte un revêtement isolant.

12. Appareil selon l'une des revendications 1 à 11, **caractérisé en ce que** la tête de mesure (5), pour un étalonnage intermédiaire, peut être déplacée parallèlement à la fente de mesure (7, 8) et orthogonalement à la plaque de fixation (6) jusqu'à ce que le produit à vérifier (1) se trouve à l'extérieur de la fente de mesure (8).

13. Appareil selon l'une des revendications 1 à 12, **caractérisé en ce qu'** au moins l'une des électrodes-condensateurs (10, 12, 14) est remplaçable.
